# EUROPEAN PATENT SPECIFICATION

(11) **EP 4 519 458 B1**
(45) Date of publication and mention of the grant of the patent: **25.02.2026**
(21) Application number: 23721662.7
(22) Date of filing: 21.04.2023
(51) Int. Cl.: C12Q 1/6876, C12Q 1/6883

(54) **DIAGNOSTIC BIOMARKER FOR OXIDATIVE STRESS**
DIAGNOSTISCHER BIOMARKER FÜR OXIDATIVEN STRESS
BIOMARQUEUR DIAGNOSTIQUE DU STRESS OXYDATIF

(30) Priority: 03.05.2022 EP 22171354
(43) Date of publication of application: 12.03.2025
(73) Proprietor: Evonik Operations GmbH, 45128 Essen (DE)
(72) Inventor: BÖHL, Florian, 60151 Neckargemünd (DE); ROY, Suki, Singapore 574036 (SG); BOURLAND, Jennifer, Singapore 018985 (SG); NAGARAJAN, Sanjanaa, Woodlands Drive 14, 11-357 Singapore 730521 (SG)
(74) Representative: Evonik Patent Association
(86) International application number: PCT/EP2023/060484
(87) International publication number: WO 2023/213573

(56) References cited:
- US-A1- 2013 283 404

## Description

### FIELD OF THE INVENTION

The present invention relates to a method for diagnosing a cell with oxidative stress (OS). In particular, the method is capable of identifying OS in cell caused specifically by the cell's exposure to Ultraviolet light and/or caused by ageing of the cell by determining differential methylation that takes place on CpG sites located on a particular gene body and the regulatory region of the specific gene.

### BACKGROUND OF THE INVENTION

Living organisms are subjected continuously to a variety of stresses from the outside environment. In order to resist such stresses, they maintain their homeostasis by various regulatory systems. Oxidative stress refers to a serious imbalance between the levels of reactive oxygen species (ROS) in a cell and its antioxidant defense mechanism. In order to survive this stress, living organisms have a system called redox regulation to cope with the stress to maintain their homeostasis by regulating the redox state. This system functions to adapt to many external stress agents such as radiation, ultraviolet (UV) light rays, environmental pollutants, high fever, low temperature, hypoxic condition, and infectious diseases as well as to oxidative stress from lifestyle-related diseases such as cancer, diabetes, arteriosclerosis, hypertension and obesity. However, if this regulation mechanism is broken for some reason or other, oxidative stress (OS) occurs. OS can lead to cellular damage, DNA fragmentation, apoptosis and cell death. Early detection of OS can prevent further damage in the living organism causing the organism to receive early treatment or start using protection.

There are some methods known in the art for early detection of OS. However, none of these methods known in the art have been officially used to detect OS in a cell by using a genomic sample of the body.

The human skin is constantly exposed to oxidative stress and free radicals, such as to high quantities of ROS, derived not only from ordinary metabolic reactions but also continuous exposure to air, radiation and UV rays, environmental pollutants, as well as physical and/or chemical agents (e.g., cosmetics). Under some conditions, the production of ROS may become so great that it may contribute to the pathogenesis of, for example, psoriasis or skin cancer. Oxidative damage caused by free radicals such as ROS is also a main cause of physical ageing in general, and of the skin in particular. Accordingly, there is a need in the art for detection of OS in cells, for example skin cells to prevent further damage to the cells.

Document US2013/283404 discloses that there is a correlation between oxidative stress and changes in methylation pattern of genes.

### DESCRIPTION OF THE INVENTION

The present invention attempts to solve the problems above by providing a gene biomarker, differential methylation of which, is capable of being used for detecting OS in a cell.

Since environmental factors/ agents such as, UV light exposure, ageing, diet and the like, may trigger OS which can further induce an alteration in the promoter CpG methylation status by recruiting DNA methyltransferases (DNMTs) and TET enzymes to various promoters, a biomarker resulting in differential methylation in a cell with OS is essential to overcome the problems mentioned above. In particular, the biomarker for detecting OS in a cell is differential methylation of a gene Protein Tyrosine Phosphatase Receptor Type N2 (*PTPRN2*)*.* The CpGs of *PTPRN2* in a cell with OS are differentially methylated (i.e. hypomethylated or hypermethylated) compared to the corresponding CpGs in a cell without OS. In particular, the CpGs of *PTPRN2* are differentially methylated based on the source of OS. Accordingly, *PTPRN2* may be effectively used to determine if a cell has OS. Different external agents can cause OS in a cell and the cell may have a different DNA methylation signature for each external agent causing OS. More in particular, OS in a cell caused by UV light exposure and/or ageing may result in a specific group of CpGs that are differentially methylated compared to cells which do not have OS and/or cells which do not have OS caused by UV light exposure and/or ageing. Even more in particular, the group of CpGs are one or more pre-selected CpG sites which are differentially methylated in cells with OS that is caused specifically by UV light exposure and/or ageing compared to cells with no OS or cells with OS caused by another external agent. This is particularly advantageous as using epigenetics provides a means of predicting the onset of OS in a cell, thus allowing OS to be treated earlier before causing even more damage to the cell. Further, an epigenetic marker is a long-term biomarker, that is to say it is inheritable and can be used to detect OS in the next generation as well if need be.

According to one aspect of the present invention, there is provided a method of identifying oxidative stress (OS) caused by ageing and/or Ultraviolet (UV) light exposure in a test cell, comprising
(a) determining the methylation status of at least one CpG site in Protein Tyrosine Phosphatase Receptor Type N2 (*PTPRN2*)*,* and/or the regulatory region of *PTPRN2* in a DNA sample obtained from the test cell,
(b) comparing the methylation status of the CpG site of *PTPRN2* from (a) with that of a control without OS caused by ageing and/or UV light exposure,
wherein a difference in the methylation status of the CpG site of *PTPRN2,* and/or the regulatory region of *PTPRN2* in the test cell compared to the CpG site in the control is indicative of the test cell having OS caused by exposure of the cell to UV light and/or ageing.

As used herein, the term "cell" refers to an intact live cell, naturally occurring or modified. The cell may be isolated from other cells, mixed with other cells in a culture, or within a tissue (partial or intact), or an organism. In particular, the cell may be a eukaryote cell. More in particular, the cell may be mammalian cell. The term "mammalian cell" refers to any cell derived from a mammalian subject. The cell may also be a cell derived from the culture and expansion of a cell obtained from a subject. The cell may also have been genetically modified to express a recombinant protein and/or nucleic acid. The mammalian cell may be from humans and other primates, including non-human primates such as chimpanzees and other apes and monkey species; farm animals such as cattle, sheep, pigs, goats and horses; domestic mammals such as dogs and cats; rodents such as mice, rats, rabbits, hamsters, and guinea pigs; birds, including domestic, wild and game birds such as chickens, turkeys and other gallinaceous birds, ducks, geese, and the like. In particular, the subject is a mammal. More in particular, the mammal is selected from the group consisting of a mouse, a rat, a guinea pig, a dog, a mini-pig, a human being, a cow, a sheep, a pig, a goat, a horse, a donkey, and a mule. In particular, the mammalian cell may be a skin cell, a stem cell or a cell derived therefrom. More in particular, the mammalian cell may be a skin cell.

As used herein, a "CpG site" or "methylation site" is a nucleotide within a nucleic acid (DNA or RNA) that is susceptible to methylation either by natural occurring events *in vivo* or by an event instituted to chemically methylate the nucleotide *in vitro.* For example, in *PTPRN2* there may be about 1300 CpG sites. Some of these sites may be hypermethylated and some may be hypomethylated in a cell with OS compared to a cell with no OS. In particular, the methylation status of at least one CpG site selected from the following list of 8 CpG sites in *PTPRN2* is determined to determine if the cell has OS caused by ageing and/or UV light exposure:

**Table 1. 8 CpG sites in PTPRN2 that are differentially methylated in a cell with OS caused by ageing and/or UV light exposure**

| **No.** | **CpG site** | **chr** | **pos** |
|---|---|---|---|
| 1 | cg11991903 | chr7 | 157794519 |
| 2 | cg17530002 | chr7 | 157701262 |
| 3 | cg25330725 | chr7 | 158180337 |
| 4 | cg21459816 | chr7 | 157588756 |
| 5 | cg03399048 | chr7 | 158180335 |
| 6 | cg26059287 | chr7 | 157468443 |
| 7 | cg05416758 | chr7 | 157783082 |
| 8 | cg21380084 | chr7 | 157937891 |

is determined to determine if the cell has OS caused by ageing and/or UV light exposure:

As used herein, a "methylated nucleic acid molecule" refers to a nucleic acid molecule that contains one or more nucleotides that is/are methylated.

A "CpG island" as used herein describes a segment of DNA sequence that comprises a functionally or structurally deviated CpG density. For example, Yamada et al. have described a set of standards for determining a CpG island: it must be at least 400 nucleotides in length, has a greater than 50% GC content, and an OCF/ECF ratio greater than 0.6 (Yamada et al., 2004, Genome Research, 14, 247-266). Others have defined a CpG island less stringently as a sequence at least 200 nucleotides in length, having a greater than 50% GC content, and an OCF/ECF ratio greater than 0.6 (Takai et al., 2002, Proc. Natl. Acad. Sci. USA, 99, 3740-3745). In context of the present invention, the terms "methylation profile", "methylation pattern", "methylation state" or "methylation status," are used herein to describe the state, situation or condition of methylation of a genomic sequence, and such terms refer to the characteristics of a DNA segment at a particular genomic locus in relation to methylation. Such characteristics include, but are not limited to, whether any of the cytosine (C) residues within this DNA sequence are methylated, location of methylated C residue(s), percentage of methylated C at any particular stretch of residues, and allelic differences in methylation due to, e.g., difference in the origin of the alleles.

The term "methylation status" refers to the status of a specific methylation site (i.e. methylated vs. non-methylated) which means a residue or methylation site is methylated or not methylated. Then, based on the methylation status of one or more methylation sites, a methylation profile may be determined. Accordingly, the term "methylation profile" or also "methylation pattern" refers to the relative or absolute concentration of methylated C residues or unmethylated C residues at any particular stretch of residues in the genomic material of a biological sample. For example, if cytosine (C) residue(s) not typically methylated within a DNA sequence are methylated, it may be referred to as "hypermethylated"; whereas if cytosine (C) residue(s) typically methylated within a DNA sequence are not methylated, it may be referred to as "hypomethylated". Likewise, if the cytosine (C) residue(s) within a DNA sequence (e.g., the DNA from a sample nucleic acid from a test subject) are methylated as compared to another sequence from a different region or from a different individual (e.g., relative to normal nucleic acid or to the standard nucleic acid of the reference sequence), that sequence is considered hypermethylated compared to the other sequence. Alternatively, if the cytosine (C) residue(s) within a DNA sequence are not methylated as compared to another sequence from a different region or from a different individual, that sequence is considered hypomethylated compared to the other sequence. These sequences are said to be "differentially methylated". Measurement of the levels of differential methylation may be done by a variety of ways known to those skilled in the art. One method is to measure the methylation level of individual interrogated CpG sites determined by the bisulfite sequencing method, as a non-limiting example.

As used herein, a "methylated nucleotide" or a "methylated nucleotide base" refers to the presence of a methyl moiety on a nucleotide base, where the methyl moiety is usually not present in a recognized typical nucleotide base. For example, cytosine in its usual form does not contain a methyl moiety on its pyrimidine ring, but 5-methylcytosine contains a methyl moiety at position 5 of its pyrimidine ring. Therefore, cytosine in its usual form may not be considered a methylated nucleotide and 5-methylcytosine may be considered a methylated nucleotide. In another example, thymine may contain a methyl moiety at position 5 of its pyrimidine ring, however, for purposes herein, thymine may not be considered a methylated nucleotide when present in DNA. Typical nucleotide bases for DNA are thymine, adenine, cytosine and guanine. Typical bases for RNA are uracil, adenine, cytosine and guanine. Correspondingly a "methylation site" is the location in the target gene nucleic acid region where methylation has the possibility of occurring. For example, a location containing CpG is a methylation site wherein the cytosine may or may not be methylated. In particular, the term "methylated nucleotide" refers to nucleotides that carry a methyl group attached to a position of a nucleotide that is accessible for methylation. These methylated nucleotides are usually found in nature and to date, methylated cytosine that occurs mostly in the context of the dinucleotide CpG, but also in the context of CpNpG- and CpNpN-sequences may be considered the most common. In principle, other naturally occurring nucleotides may also be methylated but they will not be taken into consideration with regard to any aspect of the present invention.

In context of the present invention, the terms "methylation profile", "methylation pattern", "methylation state" or "methylation status," are used herein to describe the state, situation or condition of methylation of a genomic sequence, and such terms refer to the characteristics of a DNA segment at a particular genomic locus in relation to methylation. Such characteristics include, but are not limited to, whether any of the cytosine (C) residues within this DNA sequence are methylated, location of methylated C residue(s), percentage of methylated C at any particular stretch of residues, and allelic differences in methylation due to, e.g., difference in the origin of the alleles.

The term "hypermethylation" refers to the average methylation state corresponding to an increased presence of 5-mCyt at one or a plurality of CpG dinucleotides within a DNA sequence of a test DNA sample, relative to the amount of 5-mCyt found at corresponding CpG dinucleotides within a normal control DNA sample. In particular, control refers to a cell with no indication of OS.

The term "hypomethylation" refers to the average methylation state corresponding to a decreased presence of 5-mCyt at one or a plurality of CpG dinucleotides within a DNA sequence of a test DNA sample, relative to the amount of 5-mCyt found at corresponding CpG dinucleotides within a normal control DNA sample. In particular, control refers to a cell with no indication of OS.

As used herein, the term "gene' refers to the respective genomic DNA sequence, including any promoter and regulatory sequences of the gene (e.g., enhancers and other gene sequences involved in regulating expression of the gene), and/or the body of the gene in itself. A gene sequence may be an expressed sequence (e.g., expressed RNA, mRNA, cDNA). Further, where SNPs are known within genes the term shall be taken to include all sequence variants thereof.

As used herein, the term "genomic material" refers to nucleic acid molecules or fragments of the genome of the subject or group of subjects. In particular, such nucleic acid molecules or fragments are DNA or RNA or hybrids thereof, and most preferably are molecules of the DNA genome of a subject or group of subjects.

As used herein, the "promoter" or "gene promoter" used interchangeably with the term 'regulatory region' or 'regulatory sequence' refers to the respective contiguous gene DNA sequence extending from 1.5 kb upstream to 1.5 kb downstream relative to the transcription start site (TSS), or contiguous portions thereof. In particular, 'regulatory region' refers to the respective contiguous gene DNA sequence extending from 1.5 kb upstream to 0.5 kb downstream relative to the TSS. In some examples, 'regulatory region' refers to the respective contiguous gene DNA sequence extending from 1.5 kb upstream to the downstream edge of a CpG island that overlaps with the region from 1.5 kb upstream to 1.5 kb downstream from TSS (and is such cases, my thus extend even further beyond 1.5 kb downstream), and contiguous portions thereof. In particular, with respect to *PTPRN2,* any CpG dinucleotide of the gene that is coordinately methylated with the 'regulatory region' of the gene, has substantial diagnostic/classification utility as disclosed herein.

As used herein, the "DNA sample" refers to the DNA extracted from the cell according to any aspect of the present invention using known methods in the art.

In particular, when there is differential methylation detected in a test cell, that is to say that the cell displays hypermethylation or hypomethylation at, at least one CpG site in comparison to the control (i.e., a cell without indication of OS), then the test cell has OS. More in particular, when there is differential methylation detected in one of the 8 specific CpG sites listed above in Table 1, that is to say that the cell displays hypermethylation or hypomethylation at, at least one CpG site from the list of 8 CpG sites in Table 1 comparison to the control (i.e., a cell without indication of OS or a cell with indication of OS cause by an external factor other than UV light exposure and/or ageing), then the test cell has OS caused by UV light exposure and/or ageing.

In particular, in step (a) the methylation status of at least at least 2, 3, 4, 5, 6, 7, or 8 CpG sites from Table 1 in *PTPRN2* are determined.

The method according to any aspect of the present invention, may further comprise the step of:
(i) performing bisulfite modification to the DNA sample before step (a).

'Bisulfite treatment' of genomic DNA used interchangeably with the term 'bisulfite modification', refers to the treatment of the genomic DNA with a deaminating agent such as a bisulfite that may be used to treat all DNA, methylated or not. In particular, the term "bisulfite" as used herein encompasses any suitable type of bisulfite, such as sodium bisulfite, or other chemical agents that are capable of chemically converting a cytosine (C) to an uracil (U) without chemically modifying a methylated cytosine and therefore can be used to differentially modify a DNA sequence based on the methylation status of the DNA, e.g., U.S. Pat. Pub. US 2010/0112595. As used herein, a reagent that "differentially modifies" methylated or non-methylated DNA encompasses any reagent that modifies methylated and/or unmethylated DNA in a process through which distinguishable products result from methylated and non-methylated DNA, thereby allowing the identification of the DNA methylation status. Such processes may include, but are not limited to, chemical reactions (such as a C to U conversion by bisulfite) and enzymatic treatment (such as cleavage by a methylation-dependent endonuclease). Thus, an enzyme that preferentially cleaves or digests methylated DNA is one capable of cleaving or digesting a DNA molecule at a much higher efficiency when the DNA is methylated, whereas an enzyme that preferentially cleaves or digests unmethylated DNA exhibits a significantly higher efficiency when the DNA is not methylated.

Accordingly, before step (a) according to any aspect of the present invention is carried out, the genomic DNA contained/ obtained or extracted from the cell, is first bisulfite treated.

An alternative method available in the art may be used instead of bisulfite treatment. A skilled person will understand which other methods to use. In one example, TET-assisted pyridine borane sequencing (TAPS) may be used for detection of 5mC and 5hmC (Yibin Liu, et al., Nature Biotechnology, 37: 424-429 (2019).

The cell used according to any aspect of the present invention is obtained from a biological sample selected from the group consisting of blood, brain, sperm and any other tissue or sample that provides genomic DNA to be used in the method according to any aspect of the present invention. In particular, the biological sample may comprise any biological material obtained from the subject that contains DNA, and may be liquid, solid or both, may be tissue or bone, or a body fluid such as blood, lymph, etc. In particular, the biological sample useful for the present invention may comprise biological cells or fragments thereof.

The term "test" used in conjunction with the term cell herein refers to a cell that is subjected to the method according to any aspect of the present invention and is the basis for an analysis application of the present invention. A 'test cell' is therefore a cell or a group of cells being tested according to any aspect of the present invention or a profile being obtained or generated in this context. Conversely, the term "reference" or 'control' shall denote, mostly predetermined, entities which are used for a comparison with the test entity. In particular, a 'test cell' refers to a cell being tested for OS where the methylation status has to be determined and a 'control' refers to a cell without OS where the methylation status is already known and used as a reference.

According to a further aspect of the present invention, there is provided a method of identifying oxidative stress (OS) caused by ageing and/or Ultraviolet (UV) light exposure in at least one cell, comprising detecting a difference in expression of Protein Tyrosine Phosphatase Receptor Type N2 (*PTPRN*2)*.*

The term 'epigenetic marker' as used herein refers to a gene in itself and/or CpG sites of the gene body and/or CpG sites of the regulatory region of the gene, that comprises epigenetic modifications, wherein the epigenetic modifications enable the gene to be used as a marker to determine a particular disease. In particular, the term "epigenetic marker" is defined as at least one of a DNA methylation marker, a histone modification marker, and a deacetylase marker. More in particular, the epigenetic marker for OS caused by ageing and/or Ultraviolet (UV) light exposure in a cell is the differential methylation that is found in the CpG sites on the gene body and/or regulatory region of *PTPRN2.* Even more in particular, the epigenetic marker for OS caused by ageing and/ or UV light exposure is the differential methylation of the 8CpG sites in Table 1 above which are found in the gene body and/or regulatory region of *PTPRN2* in the cell with OS caused by ageing and/ or UV light exposure relative to the control cell with no OS or with OS caused by another external factor other than ageing and/ or UV light exposure. In particular, promoter-specific hyper/hypomethylation leads to changes in the expression of different genes. More in particular, the differential methylation of the CpG sites in *PTPRN2* results in the differential expression of *PTPRN2.* This differential expression of the gene relative to a control, where there is no differential methylation, is indicative of OS, particularly, that caused by ageing and/ or UV light exposure in the cell tested. In one example, hypermethylation of the promoter is associated with the inactivation of gene expression of *PTPRN2.* In another example, the hypomethylation of the promoter is associated with the (over)activation of gene expression of *PTPRN2.*

### BRIEF DESCRIPTION OF FIGURES

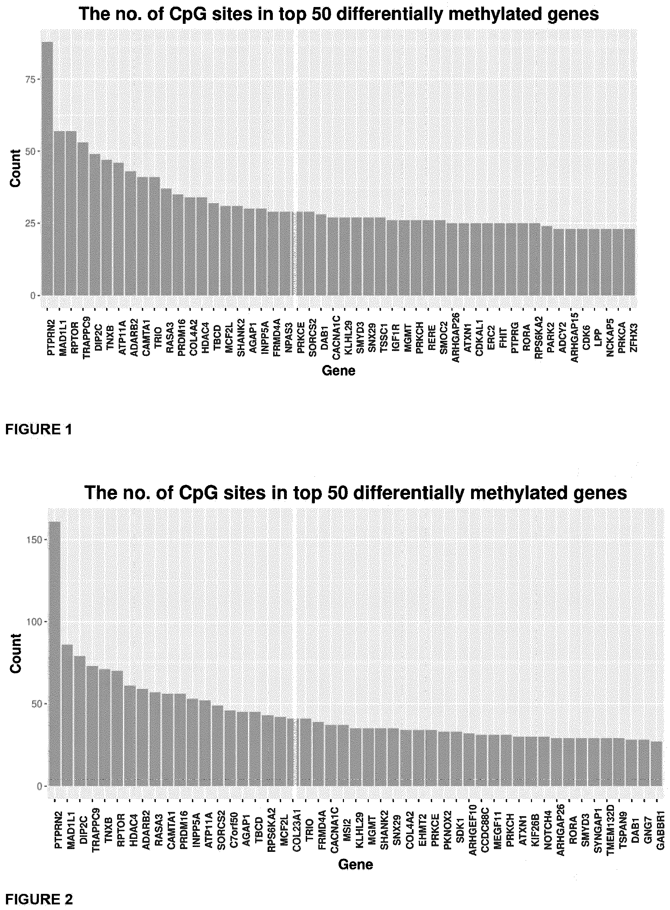

Figure 1 is a graph showing the number of probes that were differentially methylated in different gene bodies and promoter regions of cells where artificial OS (high UV light for 24hrs) was induced according to Example 1. As can be seen, genes, *MAD1L1* (meiotic spindle arrest component) and *PTPRN2* (similar to receptor-like protein tyrosine phosphatases) have the highest differentially methylated probe distribution. The other genes which were also differentially methylated in a cell with OS are shown.
Figure 2 is a graph showing the number of probes that were differentially methylated in different gene bodies and promoter regions of cells artificial OS (low UV light for 72hrs) was induced according to Example 1. As can be seen, genes, *MAD1L1* (meiotic spindle arrest component) and *PTPRN2* (similar to receptor-like protein tyrosine phosphatases) have the highest differentially methylated probe distribution. The other genes which were also differentially methylated in a cell with OS are shown.

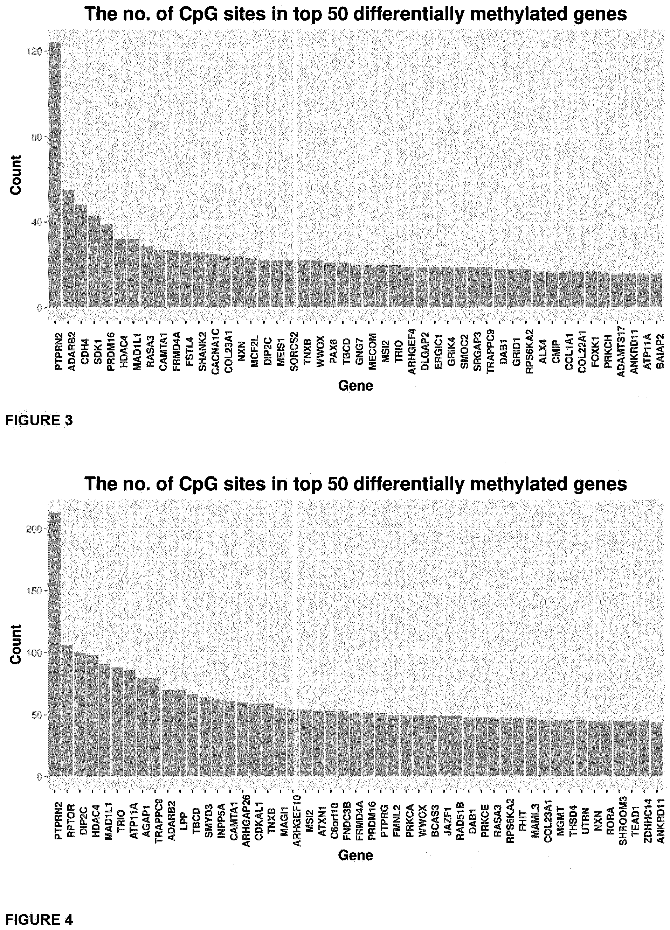

Figure 3 is a graph showing the number of probes that were differentially methylated in different gene bodies and promoter regions of cells where artificial OS (high H₂O₂ for 24hrs) was induced according to Example 2. As can be seen, genes, *MAD1L1* (meiotic spindle arrest component) and *PTPRN2* (similar to receptor-like protein tyrosine phosphatases) have the highest differentially methylated probe distribution. The other genes which were also differentially methylated in a cell with OS are shown.
Figure 4 is a graph showing the number of probes that were differentially methylated in different gene bodies and promoter regions of cells artificial OS (low H₂O₂ for 24hrs) was induced according to Example 2. As can be seen, genes, *MAD1L1* (meiotic spindle arrest component) and *PTPRN2* (similar to receptor-like protein tyrosine phosphatases) have the highest differentially methylated probe distribution. The other genes which were also differentially methylated in a cell with OS are shown.

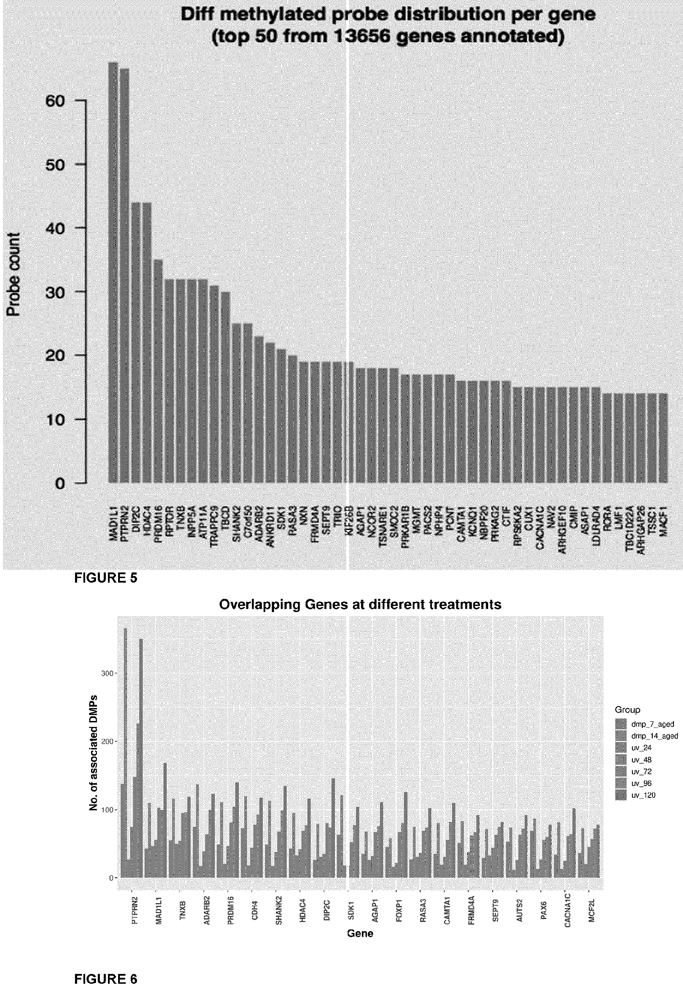

Figure 5 is a graph showing the number of probes that were differentially methylated in different gene bodies and promoter regions of cells where artificial OS according to Example 3 with Medox ^{®} in cells was induced. As can be seen, genes, *MAD1L1* (meiotic spindle arrest component) and *PTPRN2* (similar to receptor-like protein tyrosine phosphatases) have the highest differentially methylated probe distribution. The other genes which were also differentially methylated in a cell with OS are shown.
Figure 6 is a graph showing the Top 20 overlapping genes at different treatments related to UV light exposure and ageing.

### EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Example 1

### Oxidative Stress on Human Tissue with UV light

Artificial oxidative stress was induced in the cell culture system and skin tissue model to analyze the methylation status of promoters.

T-Skin models were obtained from Episkin SA, France which is composed of reconstructed human skin. Each skin model consists of a dermal equivalent overlaid by a stratified, well-differentiated epidermis derived from normal human keratinocytes. Upon receiving the skin models, it was recovered by incubating in T-Skin culture medium overnight at 37°C in a 5% CO₂ incubator.

To induce oxidative stress on a human tissue system, skin models (5x replicates) were exposed to UV light radiation (UVA 24 J/cm² + UVB 50mJ/cm²) daily for 72 hrs. Exposure to UV radiation leads to the generation of ROS which finally results in the development of oxidative stress within the cells. A control set of skin models (5x replicates) were maintained for 72hrs without any exposure to UV radiation. Followed by the treatment, skin models were collected, and genomic DNA was purified from the tissue samples using the DNeasy^{®} Blood & Tissue Kit (Qiagen). The genomic DNA was quantified using the PicroGreen^{®} or NanoDrop^{™} 2000.

The genomic DNA (500ng) from tissue samples were subjected to bisulfite conversion using the EZ DNA Methylation-Gold^{™} Kit (Zymo Research). The methylation levels were quantified using Infinium MethylationEPIC v2.0 Kit (Illumina) which can analyze over 850,000 methylation sites quantitatively across the genome at single-nucleotide resolution.

### Quality control and data processing

Methylation EPIC array data processing was performed in R version 4.1.2 (2021-11-01) using the minfi version 1.40.0. The raw intensity data (IDAT) were imported into the R (4.1.2), processed using the minfi (1.4.0) Bioconductor package,18. Quality check on samples was performed to keep probes that have a detection P-value < 0.01 in one or more samples or have a mean detection P-value < 0.05 in all samples. Then samples were normalized using functional normalization (implemented by preprocessFunnorm function in minfi) for type-bias correction and background correction.

Prior to differential methylation analysis, the probes with non-specific binding, cross reactive probes, probes affected by common SNPs, and probes annotated to the X,Y chromosomes were also filtered out. Beta-value and M-value of normalized and filtered samples were calculated using getBeta and getM function respectively, the samples were then subjected to further downstream analysis.

### Differential methylation analysis

Differential methylation analysis was performed using packages limma version 3.50.1 and DMRcate version 2.8.5. Contrast matrix was set up by comparing each corresponding treatment and control group and empirical Bayesian algorithm was used to fit the M-values based on the design and contrast model. Probes with adjusted P-value lower than 0.05 were considered as differentially methylation positions (DMPs). Annotation was performed using IlluminaHumanMethylationEPICkanno.ilmn12.hg19 and annotatr package (1.20.0).

As seen in Figure 1, one of the top genes that were differentially methylated in the promoter region is *PTPRN2* (Protein Tyrosine Phosphatase Receptor Type N2). *PTPRN2* is phosphatidylinositol phosphatase with the ability to dephosphorylate phosphatidylinositol 3-phosphate and phosphatidylinositol 4,5-diphosphate which plays important roles in lipid signaling, cell signaling and membrane trafficking.

### Example 2

### Oxidative Stress on Human Tissue with H₂O₂

Another way to induce oxidative stress on a human tissue system is through Hydrogen peroxide treatment which leads to the generation of ROS within the cells. The skin models (5x replicates) were treated with two different concentrations of Hydrogen peroxide [100µM (low) and 200µM (high)] for 2hrs and were maintained for 24hrs. A control set of skin models (5x replicates) were maintained for 24hrs without any treatment with Hydrogen peroxide. Followed by the treatment, skin models were collected, and genomic DNA was purified from the tissue samples using the DNeasy^{®} Blood & Tissue Kit (Qiagen). The genomic DNA was quantified using the PicroGreen^{®} or NanoDrop^{™} 2000.

Same method of quality control and data processing as that disclosed in Example 1 was carried out on the samples here. Further, the same differential methylation analysis as disclosed in Example 1 was carried out on the data obtained from Example 2.

The results are shown in Figures 3 and 4.

### Example 3

### Oxidative Stress on cell culture system

To investigate oxidative stress in the cell culture system, Mesenchymal Stem Cells (MSCs) were treated with Medox^{®} (Evonik, Batch:H-080719) which contains a lot of natural anthocyanins, associated with antioxidative and anti-inflammatory properties. Bone marrow derived MSCs were cultured for 1 week in Mesencult ACF Plus Medium with two doses of Medox^{®} (4x replicates): 25 µg/ml (low) and 100 µg/ml (high). The media with Medox^{®} was replaced every second day for 1 week. As a control (4x replicates), MSCs were cultured for 1 week in Mesencult ACF Plus Medium without any Medox^{®} treatment. Medox^{®} treatment is expected to produce the opposite reaction to OS.

This was followed by collection of cell pellet and genomic DNA was purified from the cell pellet using the DNeasy^{®} Blood & Tissue Kit (Qiagen). The genomic DNA was quantified using the PicroGreen^{®} or NanoDrop^{™} 2000.

The genomic DNA (500ng) from the cell pellet was subjected to bisulfite conversion using the EZ DNA Methylation-Gold^{™} Kit (Zymo Research). The methylation levels were quantified using Infinium MethylationEPIC v2.0 Kit (Illumina) which can analyze over 850,000 methylation sites quantitatively across the genome at single-nucleotide resolution.

DNA methylation profiling has been proven to be a powerful analytical tool to accurately identify the origin of tissue and the effect of environmental factors. It has several advantages as a biomarker classifier as it is a stable marker, and it can facilitate quantitative analysis at single-nucleotide resolution.

Same method of Quality control and data processing as that disclosed in Example 1 was carried out on the samples here. Further, the same differential methylation analysis as disclosed in Example 1 was carried out on the data obtained from Example 3.

As seen in Figure 5, one of the top genes that were differentially methylated in the promoter region is *PTPRN2* (Protein Tyrosine Phosphatase Receptor Type N2). *PTPRN2* is phosphatidylinositol phosphatase with the ability to dephosphorylate phosphatidylinositol 3-phosphate and phosphatidylinositol 4,5-diphosphate which plays important roles in lipid signaling, cell signaling and membrane trafficking.

### Results of both Examples 1, 2 and 3

The two independent experiment setups to analyze the methylation status of promoters during oxidative stress have shown differential methylation of *PTPRN2* promoter, suggesting it to be a potential biomarker for oxidative stress.

### Example 4

### Oxidative Stress on Human Tissue with ageing

Another way to induce oxidative stress on a human tissue system is through ageing which leads to the generation of Reactive oxygen and nitrogen species (RONS) within the cells.

The skin models were maintained in the deep well plate with media for 7 days (4x replicates), 14 days (4x replicates) and 21 days (4x replicates) respectively to induce ageing in the skin tissue. A control set of skin models (4x replicates) were collected at 0hr for genomic DNA isolation. Skin models were collected after 7 days, 14 days and 21 days respectively, and genomic DNA was purified from the tissue samples using the DNeasy^{®} Blood & Tissue Kit (Qiagen). The genomic DNA was quantified using the PicroGreen^{®} or NanoDrop^{™} 2000.

The genomic DNA (500ng) from tissue samples were subjected to bisulfite conversion using the EZ DNA Methylation-Gold^{™} Kit (Zymo Research). The methylation levels were quantified using Infinium MethylationEPIC v2.0 Kit (Illumina) which can analyze over 850,000 methylation sites quantitatively across the genome at single-nucleotide resolution.

### Oxidative Stress on Human Tissue with UV light rays

To induce oxidative stress on a human tissue system, skin models were exposed to UV radiation (UVA 24 J/cm² + UVB 50mJ/cm²) daily and cultured for 24 hrs (4x replicates), 48 hrs (4x replicates), 72hrs (4x replicates), 96hrs (4x replicates) and 120hrs (4x replicates) respectively.

Exposure to UV radiation leads to the generation of ROS which finally results in the development of oxidative stress within the cells. A control set of skin models were maintained for 24 hrs (4x replicates), 48 hrs (4x replicates), 72hrs (4x replicates), 96hrs (4x replicates) and 120hrs (4x replicates) respectively without any exposure to UV radiation. Followed by the treatment, skin models were collected, and genomic DNA was purified from the tissue samples using the DNeasy^{®} Blood & Tissue Kit (Qiagen). The genomic DNA was quantified using the PicroGreen^{®} or NanoDrop^{™} 2000.

The genomic DNA (500ng) from tissue samples were subjected to bisulfite conversion using the EZ DNA Methylation-Gold^{™} Kit (Zymo Research). The methylation levels were quantified using Infinium MethylationEPIC v2.0 Kit (Illumina) which can analyze over 850,000 methylation sites quantitatively across the genome at single-nucleotide resolution.

### Quality control and data processing

A total of 52 samples from 7 different treatments and their respective controls were analyzed (Table 2). The 7 treatments can be categorized into 2 types of oxidative stresses - UV and Age. 5 of the treatments were oxidative stress by UV at different timepoints - 24hours, 48hours, 72hours, 96hours and 120hours. Each treatment had their own respective control. The other 2 treatments were oxidative stress by aging - after 7 days and after 14 days, respectively. Both treatments were compared with the same control group. Methylation EPIC array data processing was performed in R version 4.2.2 (2021-11-10 r83330) using the minfi version 1.42.0. The raw intensity data (IDAT) were imported into R (4.2.2) and processed using the minfi (1.42.0) Bioconductor package. Quality check on samples were performed to keep probes that had a detection P-value <0.01 in one or more samples or had a mean detection P-value <0.05 in all samples. The samples were then normalized using functional normalization (implemented by preprocesssFunnorm function in minfi) for type-bias correction and background correction.

Prior to differential methylation analysis, the probes with non-specific binding, cross reactive probes, probes affected by common SNPs, and probes annotated to the X,Y chromosomes were also filtered out. Beta-value and M-value of normalized and filtered samples were calculated using getBeta and getM function respectively, the samples were then subjected to further downstream analysis.

**Table 2: Sample information for batch 2**

| **Group** | **Treatment** | **Replicates** |
|---|---|---|
| **UV Samples** | | |
| **Control** | Untreated, 24h | 4 |
| | Untreated, 48h | 4 |
| | Untreated, 72h | 4 |
| | Untreated, 96h | 4 |
| | Untreated, 120h | 4 |
| **Treatment** | Exposed to UVA 24 J/cm2 + UVB 50mJ/cm2 and cultured for 24h | 4 |
| | Exposed to UVA 24 J/cm2 + UVB 50mJ/cm2 daily and cultured for 48h | 4 |
| | Exposed to UVA 24 J/cm2 + UVB 50mJ/cm2 daily and cultured for 72h | 4 |
| | Exposed to UVA 24 J/cm2 + UVB 50mJ/cm2 daily and cultured for 96h | 4 |
| | Exposed to UVA 24 J/cm2 + UVB 50mJ/cm2 daily and cultured for 120h | 4 |

| **Aged Samples** | | |
|---|---|---|
| **Control** | Untreated, 0hr | 4 |
| **Treatment** | Untreated, cultured for 7 days in media | 4 |
| | Untreated, cultured for 14 days in media | 4 |

### Differential methylation analysis

Pair-wise differential methylation analysis (total of 7 pairs) was performed using the limma package version 3.52.4. The UV samples and Aged samples were analyzed separately. Contrast matrix was set up by comparing each corresponding treatment and control group and empirical Bayesian algorithm was used to fit the M-values based on the design and contrast model. Probes with adjusted P-value lower than 0.05 were considered as differentially methylation positions (DMPs). Annotation was performed using IlluminaHumanMethylationEPICanno.ilm10b2.hg19. Genes that overlapped at all treatments were then found and their DMPs were analyzed to identify DMPs that were present in all comparisons.

As seen in Figure 6, one of the top genes that was differentially methylated in all comparisons is *PTPRN2* (Protein Tyrosine Phosphatase Receptor Type N2). *PTPRN2* is phosphatidylinositol phosphatase with the ability to dephosphorylate phosphatidylinositol 3-phosphate and phosphatidylinositol 4,5-diphosphate which plays important roles in lipid signaling, cell signaling and membrane trafficking. *PTPRN2* gene had a total of 670 unique DMPs, of which 8 DMPs were seen in all comparisons (Table 3).

**Table 3: 8 DMPs of PTPRN2 that were common between all treatments and their methylation status at each treatment.**

| **cpg site** | **chr** | **pos** | **7_age** | **14_age** | **uv_24** | **uv_48** | **uv_72** | **uv_96** | **uv_120** |
|---|---|---|---|---|---|---|---|---|---|
| cg11991903 | chr7 | 157794519 | Hypo | Hypo | Hyper | Hyper | Hyper | Hyper | Hyper |
| cg17530002 | chr7 | 157701262 | Hypo | Hypo | Hyper | Hyper | Hyper | Hyper | Hyper |
| cg25330725 | chr7 | 158180337 | Hyper | Hyper | Hypo | Hypo | Hypo | Hypo | Hypo |
| cg21459816 | chr7 | 157588756 | Hypo | Hypo | Hyper | Hyper | Hyper | Hyper | Hyper |
| cg03399048 | chr7 | 158180335 | Hyper | Hyper | Hypo | Hypo | Hypo | Hypo | Hypo |
| cg26059287 | chr7 | 157468443 | Hypo | Hypo | Hyper | Hyper | Hyper | Hyper | Hyper |
| cg05416758 | chr7 | 157783082 | Hypo | Hypo | Hyper | Hyper | Hyper | Hyper | Hyper |
| cg21380084 | chr7 | 157937891 | Hyper | Hyper | Hypo | Hypo | Hypo | Hypo | Hypo |

## Claims

1. A method of identifying oxidative stress (OS) caused by ageing and/or Ultraviolet (UV) light exposure in a test cell, comprising
(a) determining the methylation status of at least one CpG site in Protein Tyrosine Phosphatase Receptor Type N2 (*PTPRN2*)*,* and/or the regulatory region of *PTPRN2* in a DNA sample that has been obtained from the test cell,
(b) comparing the methylation status of the CpG site of *PTPRN2* from (a) with that of a control without OS caused by ageing and/or UV light exposure,
wherein a difference in the methylation status of the CpG site of *PTPRN2,* and/or the regulatory region of *PTPRN2* in the test cell compared to the CpG site in the control is indicative of the test cell having OS caused by exposure of the cell to UV light and/or ageing.

2. The method according to claim 1, wherein in step (a) the methylation status of at least one CpG site selected from the following list of 8 CpG sites in *PTPRN2* is determined:
| **No.** | **CpG site** | **chr** | **pos** |
|---|---|---|---|
| 1 | cg11991903 | chr7 | 157794519 |
| 2 | cg17530002 | chr7 | 157701262 |
| 3 | cg25330725 | chr7 | 158180337 |
| 4 | cg21459816 | chr7 | 157588756 |
| 5 | cg03399048 | chr7 | 158180335 |
| 6 | cg26059287 | chr7 | 157468443 |
| 7 | cg05416758 | chr7 | 157783082 |
| 8 | cg21380084 | chr7 | 157937891 |

3. The method according to claim 2, wherein in step (a), the methylation status of at least 5 CpG sites from the list of 8 CpG sites in *PTPRN2* are determined.

4. The method according to either claim 2 or 3, wherein in step (a), the methylation status of all 8 CpG sites from the list of 8 CpG sites in *PTPRN2* are determined.

5. The method according to any one of the preceding claims, further comprising the step of:
(i) performing bisulfite modification to the DNA sample before step (a).

6. The method according to any one of the preceding claims, wherein the cell is obtained from a biological sample selected from the group consisting of blood, brain, sperm and any other tissue or sample that provides genomic DNA.

7. The method according to any one of the preceding claims wherein the cell is a eukaryote.

8. The method according to any one of the preceding claims, wherein the cell is from a mammal.

9. The method according to claim 8, wherein the mammal is a mouse, a rat, a guinea pig, a dog, a mini-pig, a human being, a cow, a sheep, a pig, a goat, a horse, a donkey, and a mule.

10. The method according to any one of the preceding claims, wherein the cell is a skin cell, a stem cell or a cell derived therefrom.

11. A method of identifying oxidative stress (OS) caused by ageing and/or Ultraviolet (UV) light exposure in at least one cell, the method comprising detecting a difference in expression of Protein Tyrosine Phosphatase Receptor Type N2 (*PTPRN2*) in the cell that has been obtained, as the indicator of the OS.

12. The method according claim 11, wherein the cell is obtained from a biological sample selected from the group consisting of blood, brain, sperm, skin and any other tissue or sample that provides genomic DNA.

13. The method according to either claim 11 or 12, wherein the cell is from a mammal selected from the group consisting of is a mouse, a rat, a guinea pig, a dog, a mini-pig, a human being, a cow, a sheep, a pig, a goat, a horse, a donkey, and a mule.

14. The method according to any one of claims 11 to 13, wherein the cell is a skin cell, stem cell or a cell derived therefrom.

## Patentansprüche

1. Verfahren zum Identifizieren von durch Altern und/oder Aussetzung zu ultraviolettem (UV) Licht verursachtem oxidativem Stress (OS), umfassend
(a) Bestimmen des Methylierungszustands von mindestens einer CpG-Stelle in PTPRN2 (Protein Tyrosine Phosphatase Receptor Type N2) und/oder der regulatorischen Region von PTPRN2 in einer DNA-Probe, die aus der Testzelle gewonnen wurde,
(b) Vergleichen des Methylierungszustands der CpG-Stelle des PTPRN2 aus (a) mit dem einer Kontrolle ohne durch Altern und/oder Aussetzung zu UV-Licht verursachten OS,
wobei ein Unterschied im Methylierungszustand der CpG-Stelle des PTPRN2 und/oder der regulatorischen Region von PTPRN2 in der Testzelle im Vergleich zu der CpG-Stelle in der Kontrolle anzeigt, dass die Testzelle durch Altern und/oder Aussetzung zu UV-Licht verursachten OS aufweist.

2. Verfahren nach Anspruch 1, wobei in Schritt (a) der Methylierungszustand mindestens einer CpG-Stelle bestimmt wird, die aus der folgenden Liste von 8 CpG-Stellen in PTPRN2 ausgewählt wurde:
| Nr. | CpG-Stelle | Chr | Pos |
|---|---|---|---|
| 1 | cg11991903 | Chr7 | 157794519 |
| 2 | cg17530002 | Chr7 | 157701262 |
| 3 | cg25330725 | Chr7 | 158180337 |
| 4 | cg21459816 | Chr7 | 157588756 |
| 5 | cg03399048 | Chr7 | 158180335 |
| 6 | cg26059287 | Chr7 | 157468443 |
| 7 | cg05416758 | Chr7 | 157783082 |
| 8 | cg21380084 | Chr7 | 157937891 |

3. Verfahren nach Anspruch 2, wobei in Schritt (a) der Methylierungszustand von mindestens 5 CpG-Stellen aus der Liste der 8 CpG-Stellen in PTPRN2 bestimmt wird.

4. Verfahren nach entweder Anspruch 2 oder 3, wobei in Schritt (a) der Methylierungszustand von allen 8 CpG-Stellen aus der Liste von 8 CpG-Stellen in PTPRN2 bestimmt wird.

5. Verfahren nach einem der vorhergehenden Ansprüche, ferner umfassend den folgenden Schritt:
(i) Durchführen von Bisulfitmodifikation an der DNA-Probe vor Schritt (a).

6. Verfahren nach einem der vorhergehenden Ansprüche, wobei die Zelle aus einer biologischen Probe gewonnen wird, die aus der Gruppe bestehend aus Blut, Hirn, Sperma und einem anderen Gewebe oder einer anderen Probe ausgewählt wurde, das/die Genom-DNA bereitstellt.

7. Verfahren nach einem der vorhergehenden Ansprüche, wobei es sich bei der Zelle um einen Eukaryoten handelt.

8. Verfahren nach einem der vorhergehenden Ansprüche, wobei die Zelle aus einem Säugetier stammt.

9. Verfahren nach Anspruch 8, wobei es sich bei dem Säugetier um eine Maus, eine Ratte, ein Meerschweinchen, einen Hund, ein Minischwein, einen Menschen, eine Kuh, ein Schaf, ein Schwein, eine Ziege, ein Pferd, einen Esel bzw. ein Maultier handelt.

10. Verfahren nach einem der vorhergehenden Ansprüche, wobei es sich bei der Zelle um eine Hautzelle, eine Stammzelle oder eine daraus abgeleitete Zelle handelt.

11. Verfahren zum Identifizieren von durch Altern und/oder Aussetzung zu ultraviolettem (UV) Licht verursachtem oxidativem Stress (OS) in mindestens einer Zelle, wobei das Verfahren Nachweisen eines Unterschieds bei der Expression von PTPRN2 (Protein Tyrosine Phosphatase Receptor Type N2) in der Zelle, die gewonnen wurde, als Indikator von OS umfasst.

12. Verfahren nach Anspruch 11, wobei die Zelle aus einer biologischen Probe gewonnen wird, die aus der Gruppe bestehend aus Blut, Hirn, Sperma, Haut und einem anderen Gewebe oder einer anderen Probe ausgewählt wurde, das/die Genom-DNA bereitstellt.

13. Verfahren nach entweder Anspruch 11 oder 12, wobei die Zelle aus einem Säugetier stammt, das aus der Gruppe bestehend aus einer Maus, einer Ratte, einem Meerschweinchen, einem Hund, einem Minischwein, einem Menschen, einer Kuh, einem Schaf, einem Schwein, einer Ziege, einem Pferd, einem Esel und einem Maultier ausgewählt wurde.

14. Verfahren nach einem der Ansprüche 11 bis 13, wobei es sich bei der Zelle um eine Hautzelle, Stammzelle oder eine daraus abgeleitete Zelle handelt.

## Revendications

1. Procédé d'identification du stress oxydatif (SO) causé par le vieillissement et/ou l'exposition aux rayons ultraviolets (UV) dans une cellule d'essai, comprenant
(a) la détermination de l'état de méthylation d'au moins un site CpG dans le gène du récepteur de la protéine tyrosine phosphatase de type N2 (*PTPRN2*) et/ou la région régulatrice de *PTPRN2* dans un échantillon d'ADN qui a été obtenu à partir de la cellule d'essai ;
(b) la comparaison de l'état de méthylation du site CpG de *PTPRN2* de (a) à celui d'un témoin sans SO causé par le vieillissement et/ou l'exposition aux rayons UV,
dans lequel une différence entre l'état de méthylation du site CpG de *PTPRN2* et/ou de la région régulatrice de *PTPRN2* dans la cellule d'essai et celui du site CpG dans le témoin indique que la cellule d'essai présente un SO causé par l'exposition aux rayons UV et/ou le vieillissement.

2. Procédé selon la revendication 1, dans lequel, à l'étape (a), l'état de méthylation d'au moins un site CpG choisi dans la liste suivante de 8 sites CpG dans *PTPRN2* est déterminé :
| N° | Site CpG | chr | pos |
|---|---|---|---|
| 1 | cg11991903 | chr7 | 157794519 |
| 2 | cg17530002 | chr7 | 157701262 |
| 3 | cg25330725 | chr7 | 158180337 |
| 4 | cg21459816 | chr7 | 157588756 |
| 5 | cg03399048 | chr7 | 158180335 |
| 6 | cg26059287 | chr7 | 157468443 |
| 7 | cg05416758 | chr7 | 157783082 |
| 8 | cg21380084 | chr7 | 157937891 |

3. Procédé selon la revendication 2, dans lequel, à l'étape (a), l'état de méthylation d'au moins 5 sites CpG parmi les 8 sites CpG de *PTPRN2* est déterminé.

4. Procédé selon la revendication 2 ou 3, dans lequel, à l'étape (a), l'état de méthylation de l'ensemble des 8 sites CpG dans la liste de 8 sites CpG dans *PTPRN2* est déterminé.

5. Procédé selon l'une quelconque des revendications précédentes, comprenant en outre l'étape de :
(i) modification bisulfite de l'échantillon d'ADN avant l'étape (a).

6. Procédé selon l'une quelconque des revendications précédentes, dans lequel la cellule est obtenue à partir d'un échantillon biologique choisi dans le groupe constitué de sang, de tissu cérébral, de sperme et de tout autre tissu ou échantillon apte à fournir de l'ADN génomique.

7. Procédé selon l'une quelconque des revendications précédentes, dans lequel la cellule provient d'un eucaryote.

8. Procédé selon l'une quelconque des revendications précédentes, dans lequel la cellule provient d'un mammifère.

9. Procédé selon la revendication 8, dans lequel le mammifère est une souris, un rat, un cobaye, un chien, un miniporc, un être humain, une vache, un mouton, un porc, une chèvre, un cheval, un âne et une mule.

10. Procédé selon l'une quelconque des revendications précédentes, dans lequel la cellule est une cellule cutanée, une cellule souche ou une cellule dérivée de celles-ci.

11. Procédé d'identification du stress oxydatif (SO) causé par le vieillissement et/ou l'exposition aux rayons ultraviolets (UV) dans au moins une cellule, le procédé comprenant la détection d'une différence d'expression du gène du récepteur de la protéine tyrosine phosphatase de type N2 *(PTPRN2)* dans la cellule qui a été obtenue, en tant qu'indicateur de SO.

12. Procédé selon la revendication 11, dans lequel la cellule est obtenue à partir d'un échantillon biologique choisi dans le groupe constitué de sang, de tissu cérébral, de sperme et de tout autre tissu ou échantillon apte à fournir de l'ADN génomique.

13. Procédé selon la revendication 11 ou 12, dans lequel la cellule provient d'un mammifère choisi dans le groupe constitué par une souris, un rat, un cobaye, un chien, un miniporc, un être humain, une vache, un mouton, un porc, une chèvre, un cheval, un âne et une mule.

14. Procédé selon l'une quelconque des revendications 11 à 13, dans lequel la cellule est une cellule cutanée, une cellule souche ou une cellule dérivée de celles-ci.
